# EUROPEAN PATENT SPECIFICATION

(11) **EP 2 726 596 B1**
(45) Date of publication and mention of the grant of the patent: **14.10.2015**
(21) Application number: 12720563.1
(22) Date of filing: 03.05.2012
(51) Int. Cl.: C12M 1/00, C12M 1/24, C12M 1/34, C12M 3/00

(54) **BIOREACTOR CHAMBER**
BIOREAKTORKAMMER
CHAMBRE DE BIORÉACTEUR

(30) Priority: 16.05.2011 GB 201108165
(43) Date of publication of application: 07.05.2014
(73) Proprietor: Parker Hannifin Manufacturing Limited, Hemel Hempstead, Hertfordshire HP2 4SJ (GB); Kirkstall Limited, Sheffield, South Yorkshire S3 7HB (GB)
(72) Inventor: GORDON, Paul, Sheffield South Yorkshire S3 7HB (GB); WILKINSON, Malcolm, Sheffield South Yorkshire S3 7HB (GB); WARD, William, Sheffield South Yorkshire S3 7HB (GB); DAVIES, David, Milton Keynes Buckinghamshire MK8 0HB (GB)
(74) Representative: Neilson, Martin Mark
(86) International application number: PCT/GB2012/050961
(87) International publication number: WO 2012/156683

(56) References cited:
- WO-A-88/01605
- WO-A2-2010/013068
- US-A- 3 704 802
- US-A1- 2009 104 676
- US-A1- 2010 323 438
- US-B1- 6 576 458

## Description

The present invention relates to a bioreactor chamber assembly and in particular, although not exclusively, to a chamber assembly having at least two components that are releasably coupled together to define an internal chamber having a gas inlet and outlet and a liquid inlet and outlet to allow passage of a gas and a liquid through the chamber.

In vitro cell culturing is becoming increasingly important in pharmacology, physiology and toxicology research. Currently, a wide range of biological materials are cultured and studied in vitro including for example monolayer cell cultures, scaffold cultures, tissue slices etc.

It is conventionally understood that biological tissue, during growth in vivo, is subject to physical and chemical stimuli that, to varying degree, affect the pathological and physiological status which in turn affects the development and resulting function of the tissue. Accordingly, a number of different types of systems have been developed with the capability of reproducing, as closely as possible, those environmental conditions experienced by in vivo proliferating cells. Example bioreactors for cell culturing in vitro are described in WO 88/01605; US 6,576,458; WO 2005/123258 ; US 2009/0104676; GB 2470227; US 2010/0323438; WO 2010/013068 and WO 2010/040699;

Typically, these bioreactors comprise a single or an array of internal chambers within which the cells are grown. To simulate the in vivo conditions, means are typically provided to allow a through flow of a culture medium within the chamber. This typically involves one or more fluid inlet and outlet ports connecting the chamber interior to a fluid network or circuit for circulation of the medium. However, not all cells, in vivo are submerged within a liquid phase. For example, lung tissue is both in contact with a gas phase and a liquid phase. Accordingly, conventional bioreactor chambers fall short of simulating an in vivo environment in which cells experience a combination of dynamic gaseous and liquid phases. Additionally, conventional bioreactors are not readily assembled and disconnected. This can be problematic where a sample is environmentally sensitive and loading and/or removal from the chamber quickly is essential.

There is therefore a need for a bioreactor chamber assembly that addresses the above problem.

Accordingly, the present bioreactor is optimised to simulate in vivo conditions at a gas-liquid interface, for example being a similar environment for those cells and tissues forming part of the respiratory system. This is achieved, in part, by providing both gas and liquid inlets and outlets at the chamber interior to allow a through flow of both gaseous and liquid phases through the chamber interior and in contact with the sample. Additionally, the chamber interior is configured to mount and suspend the biological species (sample) at the required position to coincide with the gas-liquid interface. As such, a lower part of the sample is capable of being submerged within the liquid phase culture medium flowing through the chamber whilst an upper region of the sample may be positioned for exposure to the through flow of gas. By consideration of the relative diameters of the gas and liquid inlets and outlets and their respective positioning, a bioreactor is provided that is optimised for studying cells in vitro and tissue culturing at the region of a gas-liquid interface where both a liquid and gas phase medium flow or circulate in contact with the sample.

Additionally, the inventors provide a bioreactor chamber assembly having a convenient releasable interlocking connection mechanism to couple the bioreactor components quickly and conveniently whilst providing a reliable fluid tight seal about the chamber interior. This is achieved, in part, by constructing the chamber assembly from at least two components, with each component comprising respective interengaging formations that provide a releasable lock arrangement via, for example, a twist-lock rotation of the first and/or second components.

According to a first aspect of the present invention that is provided a bioreactor chamber assembly comprising: at least a base and a cap configured to be coupled together to define a chamber body having a wall that defines an internal chamber to accommodate a biological sample; characterised in that: the base and cap each comprising a plurality of respective interengaging flanges to allow the base and cap to be releasably coupled and locked together axially relative to a longitudinal axis bisecting the base and cap by rotation of at least one of the base and cap about the longitudinal axis; the base and cap each further comprising a respective sealing surface that may be drawn and mated together axially via an interference fit arrangement by said rotation to provide a fluid tight seal when the base and cap are locked together axially; a liquid inlet to allow a liquid to flow into the internal chamber; a liquid outlet to allow a liquid to flow out from the internal chamber and being separate to the liquid inlet; a gas inlet to allow a gas to flow into the internal chamber; and a gas outlet to allow a gas to flow out from the internal chamber and being separate to the gas inlet; wherein the liquid inlet and outlet are positioned at an axial height lower than an axial height of the gas inlet and outlet relative to a longitudinal axis of the chamber assembly extending through the base and cap such that a gas-liquid interface may be created within the internal chamber between the liquid and the gas; wherein the liquid inlet and outlet are positioned at substantially the same axial height at the chamber wall to assist maintaining the axial position of the gas-liquid interface; the chamber assembly further comprising at least one sample support mount provided at a region of the chamber wall to mount the biological sample at a position relative to the longitudinal axial such that the biological sample is maintainable in contact with the gas-liquid interface.

Preferably, the base comprises a foot portion to support the chamber body in an upright orientation when standing upon a support structure. Preferably, the cap comprises a window to allow the internal chamber to be viewed externally.

Preferably, the interengaging flanges project radially outward from at least the first and second components to allow the first and second components to be releasably coupled together axially relative to a longitudinal axis bisecting the first and second components by rotation of at least one of the first and second components about the longitudinal axis. Optionally, the respective interengaging flanges each comprise a flange portion extending in a circumferential direction around the first and second components wherein respective flange portions of the base and cap are configured to slide over one another to couple the base and cap and prevent axial separation. Optionally, each flange of the base and cap comprises a respective abutment surfaces extending in the circumferential direction around the longitudinal axis and configured to cooperatively abut one another when the base and cap are coupled together by rotation about the longitudinal axis; wherein at least one of the abutment surfaces extends in a circumferential direction at an angle transverse to a plane perpendicular to the longitudinal axis such that as the flanges of the base and cap are slid over one another the base and cap are drawn together axially.

Preferably, the base and cap each comprise a respective sealing surface to mate together to provide a fluid tight seal when the base and cap are coupled together.

Preferably, the gas inlet and outlet are positioned at substantially different axial positions at the chamber wall relative to a longitudinal axis of the chamber assembly. Optionally, a cross sectional size of the liquid outlet is greater than a cross sectional size of the liquid inlet at the chamber walls so as to provide full control of the flow rate and volume of liquid and/or gas flowing through the internal chamber, the liquid inlet and outlet are separate through pores or apertures in the chamber wall and similarly the gas inlets and outlets are separate apertures or through bores through the chamber walls. Accordingly, the bioreactor comprises at least four apertures or bores for the passage of a fluid into and from the internal chamber. Preferably, the respective inlet and outlets are positioned diametrically opposed. However, according to further specific implementations, the separate apertures maybe positioned at any radial distribution at the chamber wall.

Preferably, the assembly comprises a plurality of sample support mounts provided at different axial positions at the internal side of the chamber walls relative to a longitudinal axis of the chamber assembly. Optionally, at least one of the sample support mounts is positioned at an upper region of the chamber assembly, when the assembly is orientated in normal use. Optionally, at least one sample support mount is positioned towards a lower region of the assembly when the assembly is orientated in normal use.

Advantageously, the assembly is configured by a relative axial positioning of the liquid and gas inlets and outlets relative to a longitudinal axis of the assembly to generate a gas-liquid interface at a region between the liquid inlet and outlet and the gas inlet and outlet relative to the longitudinal axis of the assembly.

According to a second aspect of the present invention there is provided bioreactor apparatus comprising a chamber assembly as described herein: a first fluid network coupled in fluid communication to the liquid inlet and outlet, the first fluid network comprising a liquid source and a pump to supply liquid to the internal chamber from the liquid source via the liquid inlet and to exit the internal chamber via the liquid outlet.

Preferably, the bioreactor apparatus further comprises a second fluid network coupled in fluid communication to the gas inlet and outlet, the second fluid network comprising a gas source such that gas may be supplied to the internal chamber via the gas inlet and to exit the internal chamber via the gas outlet. Preferably, the apparatus further comprises a second pump to supply gas to the internal chamber via the gas inlet and to allow gas to exit the internal chamber via the gas outlet.

According to a third aspect of the present invention there is provided a method of creating and maintaining an environment to support a biological species, the method comprising: providing a chamber body having walls that define an internal chamber to accommodate the biological species, the chamber body comprising at least a base and a cap configured to be coupled together to define the chamber body; characterised in that: the base and cap each comprising a plurality of respective interengaging flanges to allow the base and cap to be releasably coupled together axially relative to a longitudinal axis bisecting the base and cap by rotation of at least one of the base and cap about the longitudinal axis, the base and cap each further comprising a respective sealing surface that may be drawn and mated together axially via an interference fit arrangement by said rotation to provide a fluid tight seal when the base and cap are coupled together axially; providing a flow of a liquid through the internal chamber in contact with biological species via a liquid inlet and a separate liquid outlet at the chamber body, the liquid inlet and outlet being positioned at substantially the same axial height at the chamber wall relative to a longitudinal axis of the chamber assembly extending through the base and cap; providing a flow of a gas through the internal chamber in contact with biological species via a gas inlet and a separate gas outlet at the chamber body; creating a gas-liquid interface within the internal chamber as the liquid inlet and outlet are positioned at an axial height lower than an axial height of the gas inlet and outlet relative to the longitudinal axis; mounting the biological species at a position relative to the longitudinal axial such that the biological sample is maintained at a region of the gas-liquid interface via at least one sample support mount provided at a region of the chamber wall; wherein the axial position of the liquid inlet and outlet assists with maintaining the position of the gas-liquid interface within the internal chamber relative to the longitudinal axial.

The present method is configured to support the growth, culturing and development of biological tissue and the growth and in particular the proliferation of living cells.
Preferably, the method further comprises maintaining and controlling the flow of the liquid through the internal chamber in contact with biological species by pumping the liquid from a liquid reservoir through the internal chamber using a pump and a suitable liquid conduit network coupled to the liquid inlet and liquid outlet. Preferably, the method further comprises maintaining and controlling the flow of the gas through the internal chamber in contact with biological species by pumping the gas from a gas reservoir through the internal chamber using a pump and a suitable gas conduit network coupled to the gas inlet and liquid outlet.

Embodiments of the invention will now be described, by way of example only and with reference to the accompanying figures in which:
figure 1 is a perspective view of a bioreactor chamber formed from a first component (cap) and a second component (base) releasably coupled together via interengaging formations and a twist-lock rotation assembly according to a specific implementation of the present invention;
figure 2 is a perspective view of the second (base) component of the bioreactor assembly of figure 1;
figure 3 is a perspective view from above of the first (cap) component of the bioreactor chamber assembly of figure 1;
figure 4 is a perspective view from below of the first (cap) component of figure 3;
figure 5 is a cross section elevation view of the bioreactor chamber interior with a sample suspended within the chamber interior at a gas-liquid interface according to a specific implementation of the present invention;
figure 6 illustrates schematically a bioreactor chamber assembly coupled to appropriate peristaltic pumps and respective liquid and gas sources for circulation of a liquid and a gas through the chamber interior according to a specific implementation of the present invention;
figure 7A illustrates a further specific implementation of the present bioreactor chamber comprising a chamber body with means for releasably coupling two respective end components to form a chamber assembly via interengaging twist-lock formations;
figure 7B illustrates the bioreactor chamber body of figure 7A releasably coupled to two end components by interengaging twist-lock formations to form a chamber assembly.

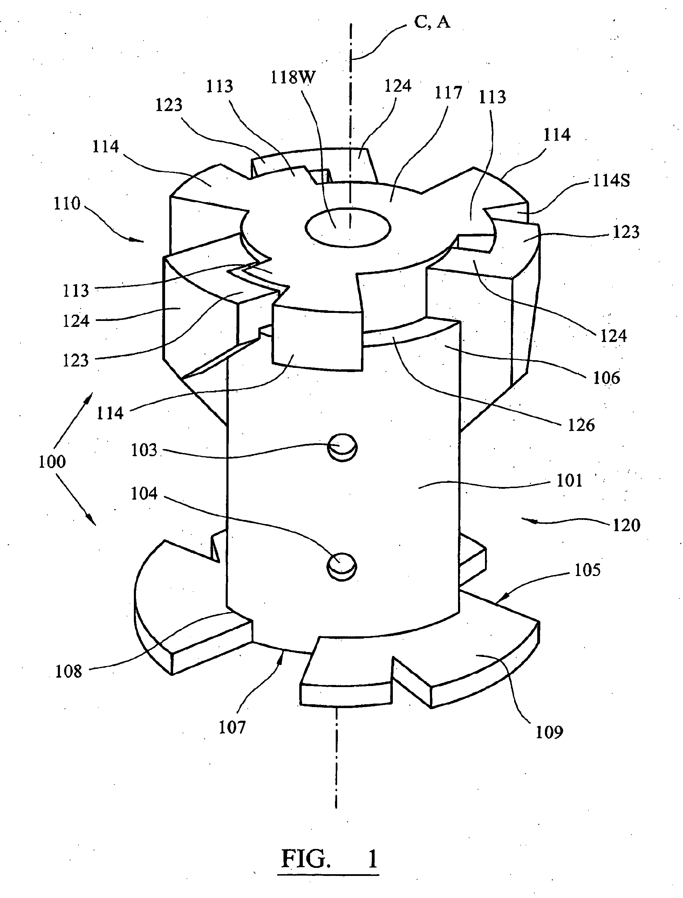

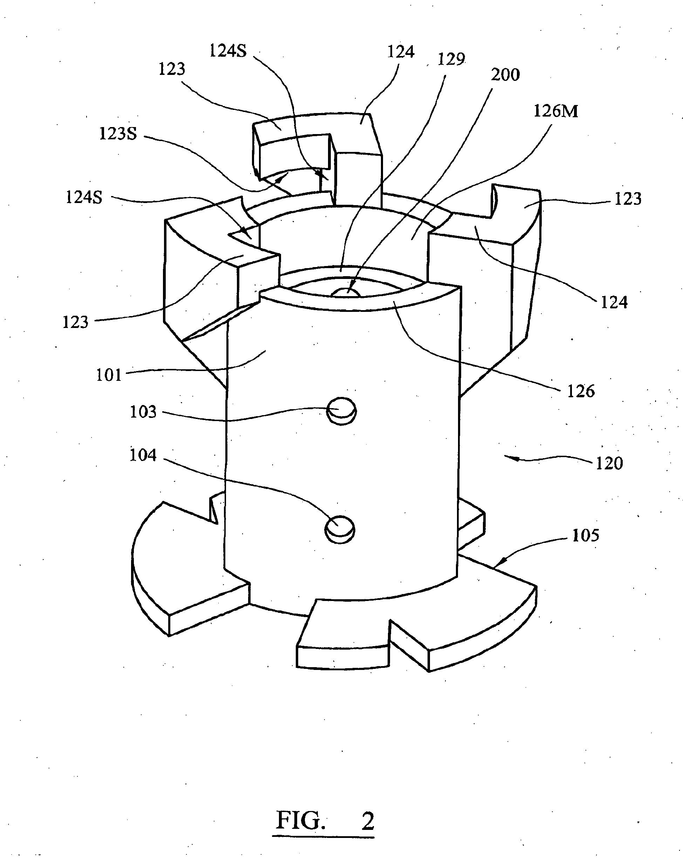

Figures 1 to 4 illustrate components of a bioreactor chamber assembly 100 according to a specific embodiment of the present invention. The assembly 100 comprises a cap component 110 as shown in figure 1, 3 and 4 and a base component 120 as shown in figure 1 and 2. Base 120 is configured for mounting upon a substantially flat horizontal surface such as a bench top. Stability for the bioreactor is provided by a foot plate 105 positioned at a lower (second end) region 108 of base 120. Foot 105 comprises projections 109 extending radially outward from base 120 relative to a longitudinal axis A centrally bisecting cap 110 and base 120.

Base 120 comprises a substantially cylindrical body 101 having a first end 106 intended to be positioned uppermost with respect to second end 108. Accordingly, base 120 is configured for positioning upon a support surface via lower surface 107.

Body 101 comprises a first pair of apertures 103 that provide a through bore into the hollow interior of body 101. Body 101 further comprises a second pair of apertures 104 that also extend through the walls of body 101 to the internal chamber of the bioreactor 100. Apertures 103 represent respective gas inlets and outlets and apertures 104 correspond to liquid inlets and outlets. Relative to longitudinal axis A, the gas inlet and outlet 103 are positioned higher than liquid inlet and outlet 104 such that apertures 103 are positioned closest to first end 106 whilst apertures 104 are positioned closest to second end 108.

Body 101 comprises substantially cylindrical chamber walls 126 that define a substantially cylindrical internal chamber 200 that extends from second end 108 to first end 106 at base 120.

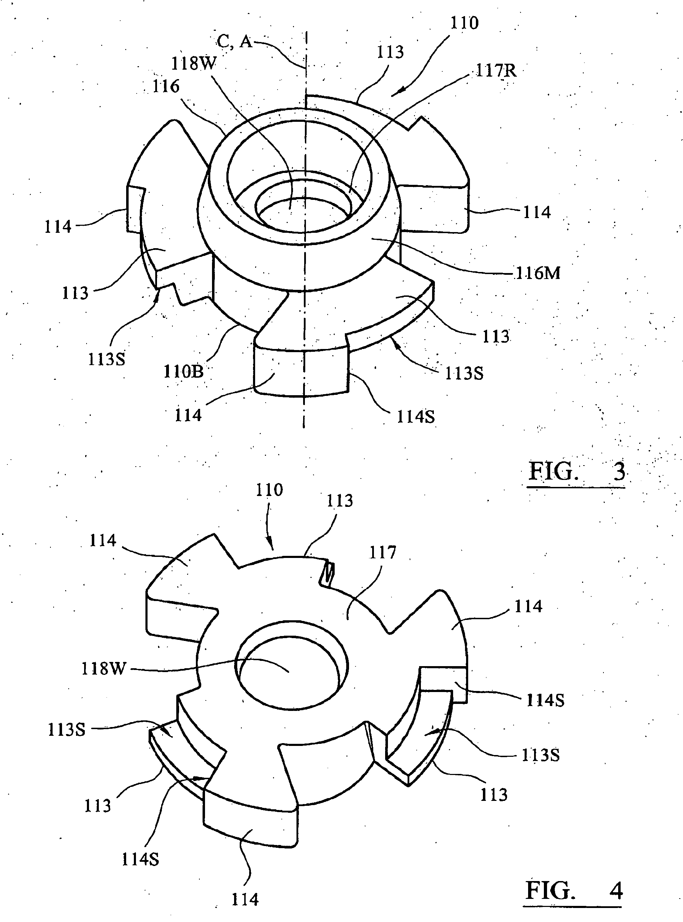

Referring to figure 1, 3 and 4, the cap 110 comprises a substantially cylindrical basal portion 110B from which three interengaging formations 114 protrude in a generally radial direction relative to axis A. The formations 114 are spaced circumferentially about the basal portion 1 10B at intervals and are substantially equidistant from one another. The formations 114 are provided with flange portions 113 that protrude from the formations 114 in a common circumferential direction around the basal portion 110B. In the arrangement shown the flange portions protrude in a clockwise circumferential direction with respect to the view of figure 1 and 4.

The flange portions 113 may be considered to be extensions of the formations 114 around the basal portion 110B, the flange portions being of a smaller thickness in a direction parallel to axis A normal to the radial and circumferential directions of the basal portion 110B. In the present embodiment, the flange portions 113 also extend a lesser radial distance away from the basal portion 110B than do the formations 114.

Each flange 113 comprises a thickness in the longitudinal direction of axis A that decreases in the circumferential direction away from foot formations 114. That is, when orientated in normal use, a downward facing surface of flange 113 is perpendicular to axis A whilst an upward facing surface 113S is inclined so as to be aligned transverse to a plane perpendicular to longitudinal axis A. This decreasing wedge shaped profile of flange 113 cooperates with a corresponding wedge shaped flange of base component 120, discussed below, to progressively strengthen the fluid tight seal at the internal chamber. Abutment surface 113 S terminates at a buffer surface 114S provided at formations 114.

The basal portion 110B of the cap 110 has a substantially cylindrical mating portion 116 extending upwardly therefrom in an axial direction; a cylinder axis C of the mating portion coinciding with a longitudinal axis of the cap 110 and of the chamber assembly. The mating portion 116 bears a mating surface 116M being a radially outer surface thereof. The mating portion 116 is tapered such that a thickness of the mating portion 116 decreases in a direction of the mating portion 116 away from the basal portion 110B, the mating portion 116 having an outer diameter that reduces with distance from the basal portion 110B.

Referring to figures 1 and 2, base 120 and in particular body 101 comprises three arms 124 at spaced apart locations around the circumference of body 101 and in particular cylindrical walls 126. Each arm 124 projects away from the body 101 in a radial direction and comprises a flange portion 123 projecting from an upper edge thereof (with respect to a normal upright orientation of the body 120). The flange portion 123 projects from the upper edge of each arm 124 in a substantially circumferential anticlockwise direction. In particular, each flange 123 extending from arm 124 comprises a corresponding abutment surface 123S configured to abut surface 113S of face 110.

Arms 124 are spaced about the body 101 at locations corresponding to those of the formations 114 of the cap 110. Accordingly, the flanges 123 are configured to cooperate with the flanges 113 to allow the cap 110 and base 120 to be coupled together. In use, the cap 110 and base 120 components are presented to one other and slid together such that the formations 114 and arms 124 of the respective components 110, 120 are substantially coplanar. The components 110, 120 are then twisted with respect to one another whereby the respective flange portions 113, 123 slide over one another via sliding contact between surfaces 113S and 123S until leading edges of each flange portion 113, 123 abut the respective alternative buffer surface 124S of arm 124 and buffer surface 114S of formations 114.

As flange 113 is tapered circumferentially such that it becomes progressively thicker in the axial direction towards formations 114, base 120 and cap 110 are drawn together in the axial direction as the abutment surfaces 113S, 123S slide in touching contact against one another. According to further embodiments, flange 123 may also comprise a tapering thickness in the circumferential direction about longitudinal axis A. Alternatively, flange 113 may comprise a uniform thickness in the axial direction whilst flange 123 comprises a wedged profile increasing in thickness in the axial direction towards arm 124.

In some embodiments the respective flange portions 113, 123 are arranged to provide a detente to reduce a risk of inadvertent separation of the components 110, 120.

An internal surface of the body 101 is provided with a mating surface 126M between the end 106 and a shoulder 129 being a portion of the internal surface of the body 101 between the mating surface 126M and a lower extending inner sidewall of body 101 that defines the majority of internal chamber 200. The inner sidewall of chamber 200 is oriented substantially parallel to the cylinder axis C of the base 120 and is of a diameter smaller than that of the mating surface 126M at its upper end in the longitudinal direction.

The mating surface 126M of the body 101 is arranged in use to abut the corresponding tapered cylindrical mating portion 116M of the cap 110. A free end of the mating portion 116 of the cap 110 is arranged to abut the shoulder 129 of the base 120. In order to reduce a risk of fluid leakage from the assembly 100 the shoulder 129 is provided with a ridged portion at a location substantially radially midway between opposed circumferential boundaries of the shoulder 129. According to some embodiments, the bioreactor is provided with a ridged portion on the free end of the mating portion 116 instead of or in addition to the shoulder 129.

According to the preferred embodiment, cap component 110 is provided with a window 118W in an upper surface 117 to allow inspection of the internal chamber. The window 118W may for example be used for visual inspection of the specimen, for example, using a microscope and/or camera arrangement. Alternatively or in addition the window 118W may provide a port through which one or more other analytical instruments may inspect the specimen. In the embodiment of figure 1 to 4 the window 118W is recessed in a surface 117 of the cap portion 110B being an uppermost region of cap 110. The window 118W may be integrally formed with the cap 110 or be a separate component connected to the cap 110. In the embodiment of figures 1 to 4 the window 118W is integrally formed by moulding, with the window being formed from the same material as the remainder of the cap 110.

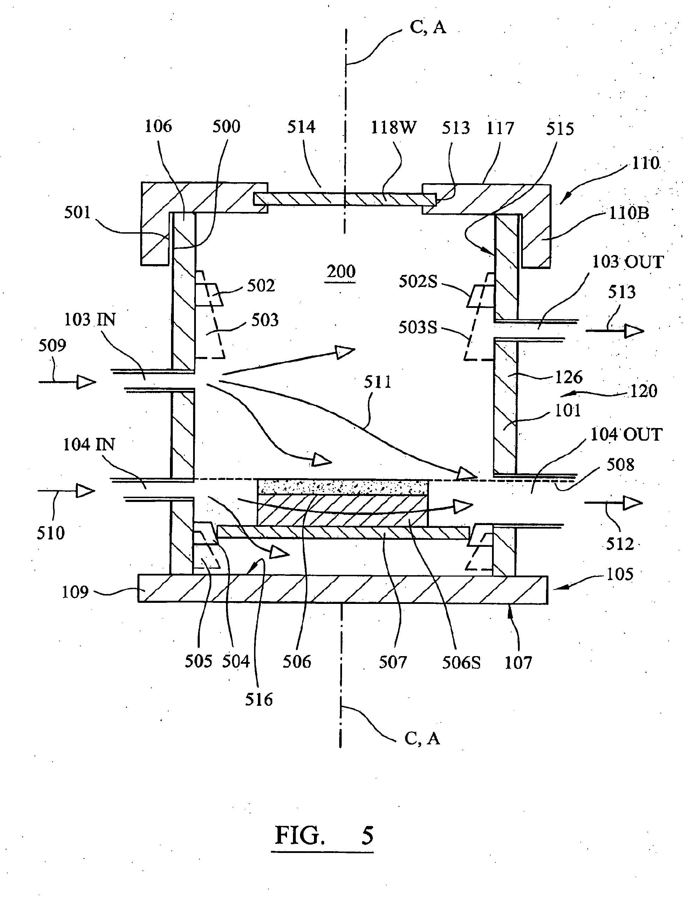

Figure 5 illustrates the assembled bioreactor of figure 1 according to a slightly different embodiment. The method of operation and the majority of the components of the bioreactors of figures 1 to 4 and figure 5 are largely the same. However, the method of attachment of lid 110 to base 120 differs slightly. In particular, and referring to figure 5, lid 110 at surface 500 comprises suitable formations to cooperate with corresponding formations provided at upper external facing surface 501 of body 101. Accordingly, lid 110 is releasably secured to base 120 via a cooperation of formations 500, 501 that may include, for example, screw threads, tongue and groove arrangements or other twist lock releasable locking mechanism. According to further embodiments, lid 110 may be secured to base 120 via snap and click members, bayonet fittings, push-fit fittings, hook and shoulder fittings and other releasable locking mechanisms operable via a push-fit or press-lock operation in the axial direction.

Additionally and referring to the embodiment of figure 5, window 118W is formed non-integrally with cap 110. In particular, cap 110 comprises a central axially aligned aperture 514. An annular pocket 513 is recessed into cap 110 and extends radially out from aperture 514 to receive and retain an outer circumferential edge of window 118W.

The chamber interior 200 is defined by cylindrical chamber walls 126 of base 120 that is enclosed at its upper end by cap 110 and at its lower end by base 105. A plurality of different sample mounts extend radially inward from the internal walls 126 of base 120. In particular, a first set 502 of sample mounts are positioned towards an upper region 106 of chamber interior 200 towards cap 110. A second set of mounts 503 may also be positioned towards upper region 106 and extend over a greater axial length than first mounts 502. Each respective set of mounts comprises a radially inward facing mounting surface 502S, 503S. Surface 502S, 503S is preferably tapered relative to the longitudinal axis A. Additionally, two further sets of sample mounts 504, 505 extend radially inward from walls 101 towards the lower region of chamber interior 200 towards base 105. Again, these further sets of mounts 504, 505 may comprise a tapering internal facing surface. This tapering or inclined surface profile 502S, 503S, provides that the bioreactor assembly 100 is configured to mount a plurality of different sized sample holders including in particular Transwell® and Thincert® mounting systems. Such holders would typically be mounted via their uppermost ends at mounts 502, 503.

Alternatively, a sample support shelf 507 maybe mounted at lower mountings 504, 505 as illustrated in figure 5. In use, a sample 506 is supported via a suitable substrate or other support structure 506S.

As shown in figure 5, gas inlet 103IN is positioned in an upper half in the axial direction through chamber walls 126. A corresponding gas outlet 103OUT is positioned above inlet 103IN and closer to the upper end 106 in the axial direction. A diameter of the gas inlet and outlet 103IN, 103OUT, is substantially the same. The respective liquid inlet 104IN and outlet 104OUT are positioned in a lower half of chamber walls 126 and are aligned approximately at the same height or distance from base 105 relative to longitudinal axis A. According to the embodiment of figure 5, a diameter of outlet 104OUT is greater than inlet 104IN. Both respective pairs of inlets 103IN, 104IN and outlets 103IN, 104OUT are positioned diametrically opposed to one another such that fluid flow from the respective inlet to the outlet passes through the internal chamber 200.

In summary, the present bioreactor 100 allows the inflow of a supply of a gas 509 via inlet 103IN. The majority of this gas then flows out of chamber interior 200 via outlet 103OUT. In addition, reactor 100 allows the inflow of a liquid 510 through inlet 104IN. The liquid flows from chamber interior 200 out through outlet 104OUT.

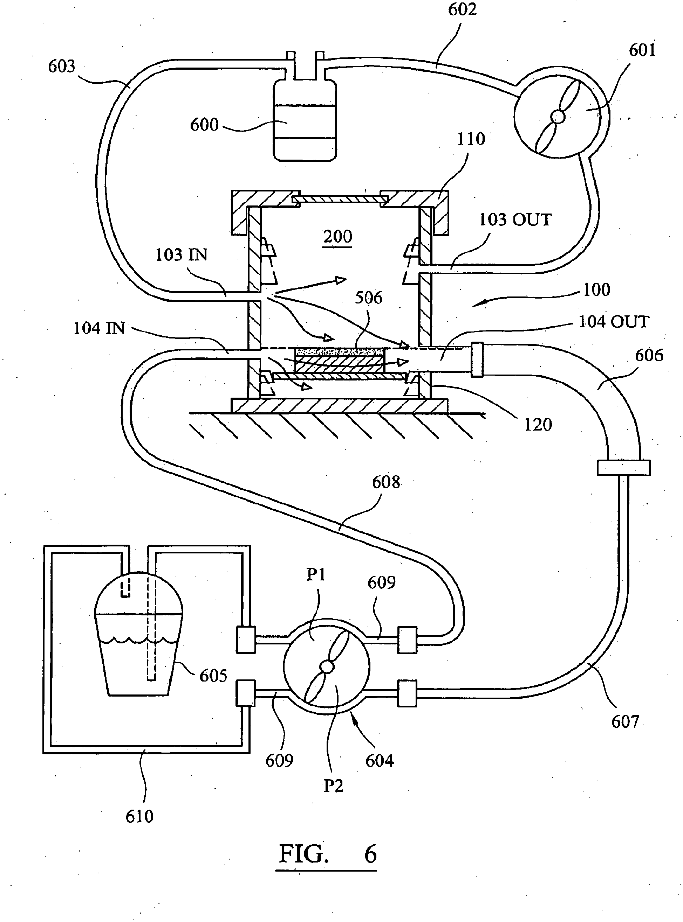

In particular, and referring to figures 5 and 6, a specimen 506 (for example a tissue culture, tissue sample or other biological material) is suspended on the specimen support 506S. The flow path 510 of liquid medium is established through the assembly 100 from the liquid inlet 104IN to the liquid outlet 104OUT. The flow rate of liquid through the inlet and outlet aperture 104IN, 104OUT is arranged to maintain a substantially stable liquid level within the assembly 100. Accordingly, a height of a gas-liquid interface 508 above a lower surface 516 of the assembly 100 is arranged to remain substantially constant.

In some embodiments this is accomplished by pumping fluid out from the assembly 100 through the liquid outlet aperture 104OUT at a higher rate than it is pumped into the assembly 100 through the liquid inlet aperture 104IN. It will be appreciated that under such conditions the liquid level 508 will typically not rise above the level of the liquid outlet aperture 104OUT. Accordingly, the chamber assembly 100 may be configured to provide a 'weir' effect, limiting a height to which the liquid level 508 may rise. If the level of the gas-liquid interface 508 in the chamber assembly 100 should rise, for example due to an unexpected influx of liquid or a temporary restriction of the outlet aperture 104OUT or an outlet hose 606, the level of the gas-liquid interface 508 will fall to and remain stable at the level previously assumed. This can be important when conducting tissue culture experiments since supporting a specimen 506 at or close to the interface 508 may be critical to simulating an environment to which a particular tissue is exposed in vivo. According to further specific embodiments, specimen 506 may be suspended or supported so as to be positioned above generally above or mainly above the gas-liquid interface 508. Accordingly, liquid flowing through chamber 200 is 'drawn-up' into specimen 506 by the action of the liquid surface tension.

As indicated, the liquid outlet aperture 104OUT is of a larger diameter than the liquid inlet aperture 104IN and the apparatus of which the assembly 100 forms part is arranged to pump liquid out from the assembly 100 at a higher rate than that at which liquid is pumped into the assembly 100. Consequently, the apparatus may pump gas out through the liquid outlet aperture 104OUT in addition to liquid. This feature has the advantage that a flow path of gas 511 through the assembly 100 may be modified and controlled by controlling the relative rates at which liquid is pumped into the chamber through the inlet aperture 104IN and fluid is pumped out through the liquid outlet aperture 104OUT. Accordingly, gas may be drawn towards and out through the liquid outlet aperture 104OUT in addition to or instead of the gas outlet aperture 103OUT.

Referring to figure 6, a peristaltic pump 604 is connected to inlet 104IN via tubing 608 and outlet 104OUT by tubing 607, 606. Pump 604 is employed to pump liquid into chamber interior 200 via inlet 104IN. In particular, chamber interior is connected to pump 604 via two sides: a pre chamber side P1 and post chamber side P2. As the flow rate is intended to be greater at the post chamber side, the flow at P2 is greater than the flow at P1. This can be controlled using a single pump 604 at a single speed such that the flow rate through the inlet and outlet tubing is different and determined by the relative diameter of tubing used. Alternatively and according to further embodiments two separate pumps 604 may be provided at the pre and post chamber side with manifold tubing of the same internal diameter but with a pump speed greater at the post chamber side (P2). The liquid network or circuit is completed as the liquid phase peristaltic pump 604 is coupled to a liquid reservoir 605 via suitable conduits 610.

In some embodiments the fluid conduits 606, 607, 609 of the second peristaltic pump P2 may be arranged to have a greater diameter than the fluid conduits 608, 609 of the first peristaltic pump P1, the first P1 and second P2 pumps having rollers arranged to rotate at different rotational speeds.

However, according to the preferred embodiment the first P1 and second P2 pumps are provided by a single pump 604 having first and second fluid conduits 609 around a common roller assembly. Thus as the rollers rotate, fluid is simultaneously pumped through both conduits 609.

In the embodiment of figure 6 the conduits 606, 608 have different diameters and therefore fluid may be pumped through the conduits at different respective rates as the pump 604 rotates at a given rotational speed.

It is to be understood that in some alternative embodiments the conduits 606, 608 have substantially the same diameter and therefore fluid is pumped through the conduits 606, 608 at substantially the same rate. In some embodiments the pump 604 is arranged to pump fluid through more than two conduits 606, 608.

The present apparatus allows a flow path of gas within the assembly 100 to be controlled so that it flows close to or in direct contact with a specimen which is particularly advantageous in experiments where exposure of tissue to gas is of particular interest or importance. Examples of such experiments include those where the specimen comprises lung tissue and/or one or more other tissues associated with a respiratory system. Experiments in respect of other biological samples may also be enhanced when performed using embodiments of the present invention.

Embodiments of the present invention provide apparatus in which experiments may be performed with specimen 506 held at or near the gas-liquid interface 508 in which a flow of liquid and/or gas is maintained over the specimen. The interface 508 may be maintained at a prescribed height within chamber 200 in a reliable manner. As described above, in some arrangements the height of the interface 508 is arranged to be corrected automatically by the apparatus in the event the height rises above the prescribed height.

Accordingly, to simulate exposure to a flow of gas at biological martial site, the present assembly is configured for connection to a gas phase network in combination with the liquid phase network as described. The gaseous fluid is circulated through chamber interior 200 by connecting inlet 103IN and outlet 103OUT to a suitable gas reservoir 600. A second or further peristaltic pump 601 is positioned between gas reservoir 600 and gas outlet 103OUT so as to draw the flow of gas 513 out of chamber interior 200. This in turn induces the flow of gas 509 into chamber interior 200. As will be appreciated, the present assembly 100 is suitable for a gas to be supplied directly to the chamber interior 200 under pressure with or without a pump 601. Where a pump 601 is included, this may be positioned at the inlet or outlet side of the chamber relative to the position within the network of the gas reservoir 600.

In some embodiments the apparatus is arranged so as to prevent the level of the interface 508 falling below that of the liquid outlet aperture 104OUT even if a flow of liquid 510 into the assembly is terminated. In some embodiments the liquid outlet aperture 104OUT is provided such that a lower level of the aperture corresponds to a lowest acceptable level of fluid in the assembly.

Other numbers of inlet and outlet apertures allowing the flow of fluid into or out from the assembly 100 than those of the embodiments illustrated are also useful. In some arrangements a chamber assembly 100 is provided with three and in some arrangements more than four apertures for allowing a flow of fluid into or out from the assembly 100.

The present invention provides a chamber assembly 100 allowing relative rapid and convenient assembly and disassembly. In some embodiments, a specimen 506 may be mounted on the specimen support 506S and the assembly 100 assembled in a rapid and reliable manner allowing a sample of biological material comprised by the specimen 506 to continue to function substantially uninterrupted whilst the assembly 100 is assembled and a supply of liquid 510 and/or gas 509 to the assembly 100 is established.

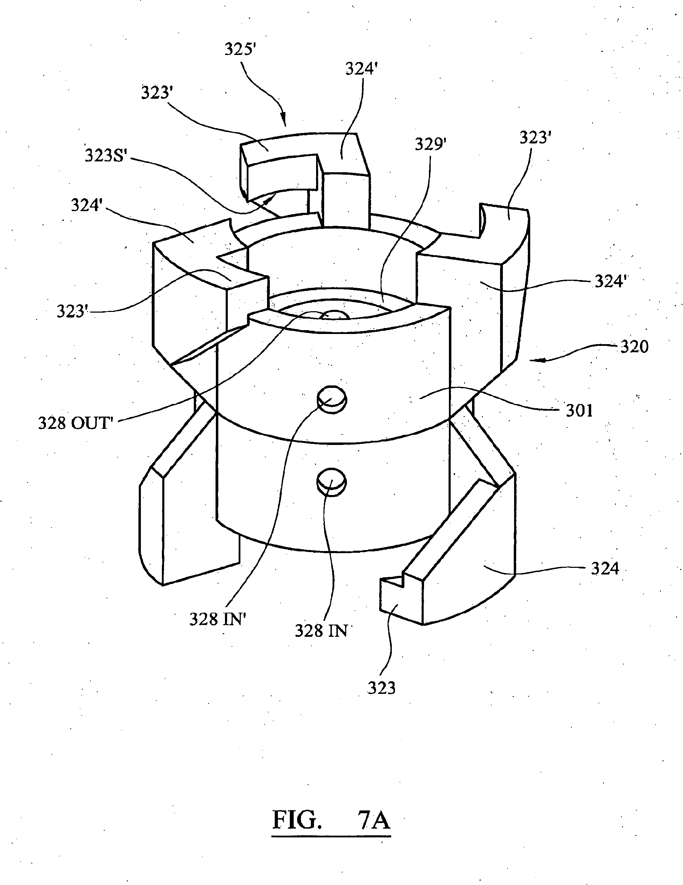

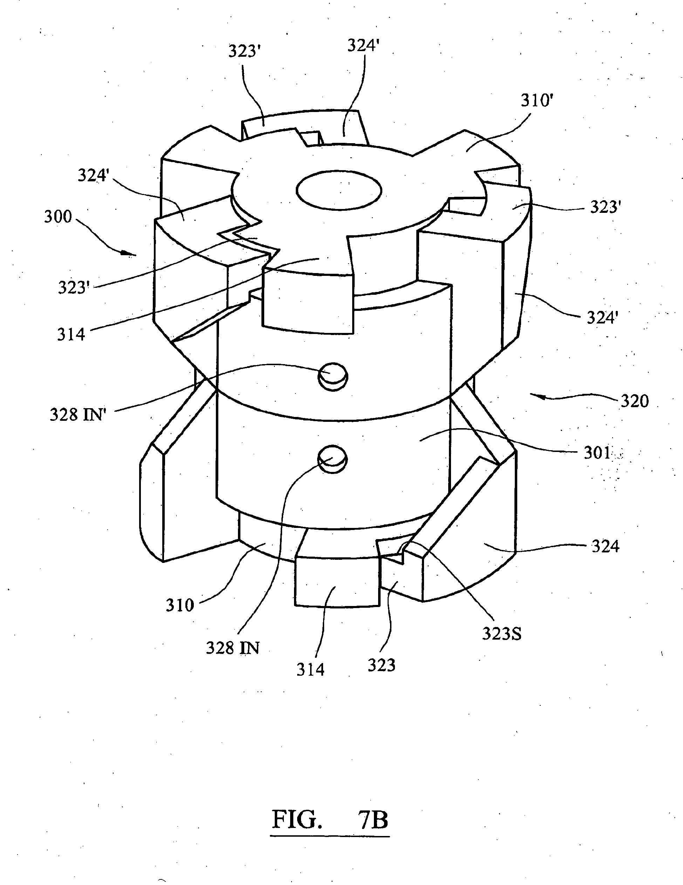

Figure 7A and 7B illustrate a bioreactor chamber assembly 300 according to a further embodiment of the present invention. As will be noted, the interengaging formations of figure 7A and 7B correspond to the interengaging formations described with reference to figures 1 to 4. The features of the embodiment of figures 7A and 7B are indicated with like reference numerals prefixed with numeral 3 instead of 1. Figure 7A shows a body component 320 only of the assembly whilst figure 7B shows the body component 320 having base (or end) components 310, 310' coupled to opposite ends thereof. The base components 310, 310' are substantially identical to one another and to the cap component 110 of the embodiment of figure 2. The base components 310, 310' are arranged to be interchangeable in the embodiment shown although other arrangements are also useful, particularly where an internal shape of the upper base component 310' is required to be different from that of the lower base component 310.

The body 320 has feet 324, 324' having tongue portions 323, 323' at opposed ends thereof defining respective jaws 325, 325' that are configured in a substantially identical manner to one another and to the formations 124 of the assemblies of figures 1 to 5. The jaws 325, 325' of the body component 320 are arranged to receive corresponding tongue portions 312,312' of the base components 310, 310' when the assembly 300 is assembled.

The body component has two pairs of apertures formed therein to allow fluid to flow through the internal volume of the body component when the base components 310, 310' are coupled to it. A gas inlet aperture 328IN' and a gas outlet aperture 328OUT' are provided at diametrically opposed locations of the body portion 320B in an upper portion of the body 320 as with the embodiment of figures 1 to 5.

A liquid inlet aperture 328IN and a liquid outlet aperture 328OUT are provided at corresponding locations below the gas inlet and outlet apertures 328IN', 328OUT'. The liquid inlet and outlet apertures 328IN, 328OUT are provided at substantially the same height. In the embodiment of figure 7A and 7B the liquid outlet aperture is arranged to have a larger diameter than the liquid inlet aperture 328IN. Other positions of the apertures 328IN, 328OUT, 328IN', 328OUT' about a circumference of the body 320 are also useful.

In some embodiments the liquid outlet aperture 328OUT is of substantially the same cross-sectional area as the liquid inlet aperture 328IN. In the present embodiment they are of substantially the same diameter. In some embodiments the liquid outlet aperture 328OUT has a diameter smaller than that of the liquid inlet aperture 328IN.

In the embodiment of figure 7A and 7B the gas inlet and outlet apertures 328IN', 328OUT' are substantially the same diameter. In some alternative embodiments the gas inlet aperture 328IN' is smaller than the gas outlet aperture 328OUT' whilst in some further embodiments the gas inlet aperture 328IN' is larger than the gas outlet aperture 328OUT'. Whilst the inlet and outlets have been described above with reference to one handling a flow of liquid and the other handling a flow of gas, it is to be understood that in some arrangements both may be used for liquid or both may be used for gas.

Referring to the embodiment of figures 7A and 7B, in some arrangements the gas inlet aperture 328IN' may be used to deliver liquid to the assembly 300 in addition to or instead of the liquid inlet aperture 328IN. The gas inlet aperture 328IN' may be used to deliver liquid continuously, or intermittently. In some arrangements the gas inlet aperture 328IN' may be used to deliver both liquid and gas substantially simultaneously.

## Claims

1. A bioreactor chamber assembly comprising:
at least a base (120) and a cap (110) configured to be coupled together to define a chamber body having a wall (126) that defines an internal chamber (200) to accommodate a biological sample;
**characterised in that**:
the base (120) and cap (110) each comprising a plurality of respective interengaging flanges (113, 123) to allow the base (120) and cap (110) to be releasably coupled and locked together axially relative to a longitudinal axis (A) bisecting the base (120) and cap (110) by rotation of at least one of the base (120) and cap (110) about the longitudinal axis (A);
the base (120) and cap (110) each further comprising a respective sealing surface (116M, 126M) that may be drawn and mated together axially via an interference fit arrangement by said rotation to provide a fluid tight seal when the base (120) and cap (110) are locked together axially;
a liquid inlet (104IN) to allow a liquid to flow into the internal chamber (200);
a liquid outlet (104OUT) to allow a liquid to flow out from the internal chamber (200) and being separate to the liquid inlet (104IN);
a gas inlet (103IN) to allow a gas to flow into the internal chamber (200); and
a gas outlet (103 OUT) to allow a gas to flow out from the internal chamber (200) and being separate to the gas inlet (103IN);
wherein the liquid inlet (104IN) and outlet (104OUT) are positioned at an axial height lower than an axial height of the gas inlet (103IN) and outlet (103OUT) relative to a longitudinal axis (A) of the chamber assembly extending through the base (120) and cap (110) such that a gas-liquid interface may be created within the internal chamber between the liquid and the gas;
wherein the liquid inlet (104IN) and outlet (104OUT) are positioned at substantially the same axial height at the chamber wall (126) to assist maintaining the axial position of the gas-liquid interface;
the chamber assembly further comprising at least one sample support mount (504) provided at a region of the chamber wall (126) to mount the biological sample at a position relative to the longitudinal axial (A) such that the biological sample is maintainable in contact with the gas-liquid interface.

2. The assembly as claimed in claim 1 wherein the base (120) comprises a foot portion (105) to support the chamber body in an upright orientation when standing upon a support structure.

3. The assembly as claimed in claim 1 or 2 wherein the cap (110) comprises a window (118W) to allow the internal chamber (200) to be viewed externally.

4. The assembly as claimed in any preceding claim wherein the interengaging flanges (113, 123) project radially outward from at least the base (120) and cap (110) to allow the base (120) and cap (110) to be releasably coupled together axially.

5. The assembly as claimed in claim 4 wherein the respective flanges (113, 123) extend in a circumferential direction around the base (120) and cap (110) wherein respective flange (113, 123) of the base (120) and cap (110) are configured to slide over one another to couple the base (120) and cap (110) and prevent axial separation.

6. The assembly as claimed in claim 5 wherein each flange (113, 123) of the base (120) and cap (110) comprises a respective abutment surfaces (113S, 123S) extending in the circumferential direction around the longitudinal axis (A) and configured to cooperatively abut one another when the base (120) and cap (110) are coupled together by rotation about the longitudinal axis (A);
wherein at least one of the abutment surfaces (113S, 123S) extends in a circumferential direction at an angle transverse to a plane perpendicular to the longitudinal axis (A) such that as the flanges (113, 123) of the base (120) and cap (110) are slid over one another the base (120) and cap (110) are drawn together axially.

7. The assembly as claimed in any preceding claim wherein the gas inlet (103IN) and outlet (103OUT) are positioned at substantially different axial height positions at the chamber wall (126) relative to the longitudinal axis (A).

8. The assembly as claimed in any preceding claim wherein a cross sectional size of the liquid outlet (104OUT) is greater than a cross sectional size of the liquid inlet (104IN) at the chamber wall (126).

9. The assembly as claimed in any preceding claim comprising a plurality of sample support mounts (502, 503, 504, 505) provided at different axial positions at the internal side of the chamber walls (126) relative to the longitudinal axis (A).

10. The assembly as claimed in claim 9 wherein at least one of the sample support mounts (502, 503, 504, 505) is positioned at an upper region of the chamber assembly, when the assembly is orientated in normal use.

11. The assembly as claimed in claims 9 or 10 wherein at least one sample support mount (504, 505) is positioned towards a lower region of the assembly when the assembly is orientated in normal use.

12. Bioreactor apparatus comprising:
a chamber assembly as claimed in any preceding claim;
a first fluid network (606,607,608) coupled in fluid communication to the liquid inlet (104IN) and outlet (104OUT), the first fluid network (606, 607, 608) comprising a liquid source (605) and a pump (604) to supply liquid to the internal chamber (200) from the liquid source (605) via the liquid inlet (104IN) and to exit the internal chamber (200) via the liquid outlet (104OUT).

13. The apparatus as claimed in claim 12 further comprising a second fluid network (602,603) coupled in fluid communication to the gas inlet (103IN) and outlet (103OUT, the second fluid network (602, 603) comprising a gas source (600) such that gas may be supplied to the internal chamber (200) via the gas inlet (103IN) and to exit the internal chamber (200) via the gas outlet (103OUT).

14. The apparatus as claimed in claim 13 further comprising a second pump (601) to supply gas to the internal chamber (200) via the gas inlet (103IN) and to allow gas to exit the internal chamber (200) via the gas outlet (103OUT).

15. A method of creating and maintaining an environment to support a biological species, the method comprising:
providing a chamber body having walls (126) that define an internal chamber (200) to accommodate the biological species, the chamber body comprising at least a base (120) and a cap (110) configured to be coupled together to define the chamber body;
**characterised in that**:
the base (120) and cap (110) each comprising a plurality of respective interengaging flanges (113, 123) to allow the base (120) and cap (110) to be releasably coupled together axially relative to a longitudinal axis (A) bisecting the base (120) and cap (110) by rotation of at least one of the base (120) and cap (110) about the longitudinal axis (A), the base (120) and cap (110) each further comprising a respective sealing surface (116M, 126M) that may be drawn and mated together axially via an interference fit arrangement by said rotation to provide a fluid tight seal when the base (120) and cap (110) are coupled together axially;
providing a flow of a liquid through the internal chamber (200) in contact with biological species via a liquid inlet (104IN) and a separate liquid outlet (104OUT) at the chamber body, the liquid inlet (104IN) and outlet (104OUT) being positioned at substantially the same axial height at the chamber wall (126) relative to a longitudinal axis (A) of the chamber assembly extending through the base (120) and cap (110);
providing a flow of a gas through the internal chamber (200) in contact with biological species via a gas inlet (103IN) and a separate gas outlet (103OUT) at the chamber body;
creating a gas-liquid interface within the internal chamber (200) as the liquid inlet (104IN) and outlet (104OUT) are positioned at an axial height lower than an axial height of the gas inlet (103IN) and outlet (103OUT) relative to the longitudinal axis (A);
mounting the biological species at a position relative to the longitudinal axial (A) such that the biological sample is maintained at a region of the gas-liquid interface via at least one sample support mount (504) provided at a region of the chamber wall (126);
wherein the axial position of the liquid inlet (104IN) and outlet (104OUT) assists with maintaining the position of the gas-liquid interface within the internal chamber (200) relative to the longitudinal axis (A).

## Patentansprüche

1. Bioreaktor-Kammer-Anordnung umfassend:
mindestens eine Basis (120) und einen Aufsatz (110), welche ausgestaltet sind, um zusammengekoppelt zu werden, um einen Kammerkörper zu definieren, welcher eine Wand (126) aufweist, was eine innere Kammer (200) definiert, um eine biologische Probe aufzunehmen;
**dadurch gekennzeichnet, dass** bzw. **gekennzeichnet durch**:
die Basis (120) und der Aufsatz (110) jeweils mehrere entsprechende in Eingriff bringbare Flansche (113, 123) umfassen, damit die Basis (120) und der Aufsatz (110) lösbar gekoppelt und zusammen axial relativ zu einer Längsachse (A), welche die Basis (120) und den Aufsatz (110) halbiert, verriegelt werden können durch eine Drehung der Basis (120) und/oder des Aufsatzes (110) um die Längsachse (A);
die Basis (120) und der Aufsatz (110) darüber hinaus eine entsprechende abdichtende Oberfläche (116M, 126M) umfassen, welche mittels einer Schnittstellen-Anpassungsanordnung **durch** die Drehung zusammengezogen und angepasst werden kann, um eine fluiddichte Dichtung bereitzustellen, wenn die Basis (120) und der Aufsatz (110) axial zusammenverriegelt sind;
einen Flüssigkeitseinlass (104IN), um einer Flüssigkeit zu ermöglichen, in die innere Kammer (200) zu fließen;
einen Flüssigkeitsauslass (104OUT), um einer Flüssigkeit zu ermöglichen, aus der inneren Kammer heraus zu fließen (200), welcher von dem Flüssigkeitseinlass (104IN) getrennt ist;
einen Gaseinlass (103IN), um einem Gas zu ermöglichen, in die innere Kammer (200) zu strömen; und
einen Gasauslass (103OUT), um einem Gas zu ermöglichen, aus der inneren Kammer (200) heraus zu strömen, welcher von dem Gaseinlass (103IN) getrennt ist;
wobei der Flüssigkeitseinlass (104IN) und der Flüssigkeitsauslass (104OUT) an einer axialen Höhe angeordnet sind, welche sich tiefer als eine axiale Höhe des Gaseinlasses (103IN) und des Gasauslasses (103OUT) relativ zu einer Längsachse (A) der Kammer-Anordnung, welche sich **durch** die Basis (120) und den Aufsatz (110) erstreckt, befindet, so dass eine Gas-Flüssigkeits-Schnittstelle in der inneren Kammer zwischen der Flüssigkeit und dem Gas erzeugt werden kann;
wobei der Flüssigkeitseinlass (104IN) und der Flüssigkeitsauslass (104OUT) an im Wesentlichen derselben axialen Höhe an der Kammerwand (126) angeordnet sind, um ein Beibehalten der axialen Position der Gas-Flüssigkeits-Schnittstelle zu unterstützen;
wobei die Kammer-Anordnung darüber hinaus mindestens eine Probenhalterung (504) umfasst, welche bei einem Bereich der Kammerwand (126) vorhanden ist, um die biologische Probe an einer Position relativ zu der Längsachse (A) zu halten, so dass die biologische Probe in Kontakt mit der Gas-Flüssigkeits-Schnittstelle haltbar ist.

2. Anordnung nach Anspruch 1, wobei die Basis (120) einen Bodenabschnitt (105) umfasst, um den Kammerkörper in einer aufrechten Ausrichtung zu halten, wenn er auf einer Haltestruktur steht.

3. Anordnung nach Anspruch 1 oder 2, wobei der Aufsatz (110) ein Fenster (118W) umfasst, um zu ermöglichen, dass die innere Kammer (200) von außen betrachtet werden kann.

4. Anordnung nach einem der vorhergehenden Ansprüche, wobei die in Eingriff bringbaren Flansche (113, 123) radial von der Basis (120) und/oder dem Aufsatz (110) nach außen ragen, um zu ermöglichen, dass die Basis (120) und der Aufsatz (110) lösbar axial zusammengekoppelt werden können.

5. Anordnung nach Anspruch 4, wobei sich die entsprechenden Flansche (113, 123) in einer Umfangsrichtung um die Basis (120) und den Aufsatz (110) herum erstrecken, wobei sich der entsprechende Flansch (113, 123) der Basis (120) und des Aufsatzes (110) ausgestaltet sind, um übereinander zu gleiten, um die Basis (120) und den Aufsatz (110) zu koppeln und eine axiale Trennung zu verhindern.

6. Anordnung nach Anspruch 5, wobei jeder Flansch (113, 123) der Basis (120) und des Aufsatzes (110) entsprechende Anstoßoberflächen (113S, 123S) umfasst, welche sich in der Umfangsrichtung um die Längsachse (A) erstrecken und ausgestaltet sind, um zusammenwirkend gegeneinander zu stoßen, wenn die Basis (120) und der Aufsatz (110) durch eine Drehung um die Längsachse (A) zusammengekoppelt werden;
wobei sich zumindest eine der Anstoßoberflächen (113S, 123S) in einer Umfangsrichtung mit einem Winkel quer zu einer Ebene senkrecht zu der Längsachse (A) erstreckt, so dass, wenn die Flansche (113, 123) der Basis (120) und des Aufsatzes (110) übereinander gleiten, die Basis (120) und der Aufsatz (110) axial zusammengezogen werden.

7. Anordnung nach einem der vorhergehenden Ansprüche, wobei der Gaseinlass (103IN) und der Gasauslass (103OUT) an im Wesentlichen unterschiedlichen axialen Höhenpositionen an der Kammerwand (126) relativ zu der Längsachse (A) angeordnet sind.

8. Anordnung nach einem der vorhergehenden Ansprüche, wobei eine Querschnittsabmessung des Flüssigkeitsauslasses (104OUT) größer als eine Querschnittsabmessung des Flüssigkeitseinlasses (104IN) an der Kammerwand (126) ist.

9. Anordnung nach einem der vorhergehenden Ansprüche, mehrere Probenhalterungen (502, 503, 504, 505) umfassend, welche an unterschiedlichen axialen Positionen an der inneren Seite der Kammerwände (126) relativ zu der Längsachse (A) vorhanden sind.

10. Anordnung nach Anspruch 9, wobei zumindest eine der Probenhalterungen (502, 503, 504, 505) an einem oberen Bereich der Kammer-Anordnung angeordnet ist, wenn die Anordnung bei einem normalen Betrieb ausgerichtet ist.

11. Anordnung nach Anspruch 9 oder 10, wobei zumindest eine Probenhalterung (504, 505) zu einem unteren Bereich der Anordnung angeordnet ist, wenn die Anordnung bei einem normalen Betrieb ausgerichtet ist.

12. Bioreaktor-Vorrichtung umfassend:
eine Kammer-Anordnung nach einem der vorhergehenden Ansprüche;
ein erstes Fluid-Netzwerk (606, 607, 608), welches in Fluidkommunikation mit dem Flüssigkeitseinlass (104IN) und dem Flüssigkeitsauslass (1040UT) gekoppelt ist, wobei das erste Fluid-Netzwerk (606, 607, 608) eine Flüssigkeitsquelle (605) und eine Pumpe (604) umfasst, um Flüssigkeit über den Flüssigkeitseinlass (104IN) von der Flüssigkeitsquelle (605) zu der inneren Kammer (200) zu führen und aus der inneren Kammer (200) über den Flüssigkeitsauslass (104OUT) auszulassen.

13. Vorrichtung nach Anspruch 12, darüber hinaus ein zweites Fluid-Netzwerk (602, 603) umfassend, welches in Fluid-Kommunikation mit dem Gaseinlass (103IN) und dem Gasauslass (103OUT) gekoppelt ist, wobei das zweite Fluid-Netzwerk (602, 603) eine Gasquelle (600) umfasst, so dass Gas über den Gaseinlass (103IN) der inneren Kammer (200) zugeführt und über den Gasauslass (103OUT) aus der inneren Kammer (200) abgelassen werden kann.

14. Vorrichtung nach Anspruch 13, darüber hinaus eine zweite Pumpe (601) umfassend, um Gas über den Gaseinlass (103IN) der inneren Kammer (200) zuzuführen und zu ermöglichen, dass Gas über den Gasauslass (103OUT) aus der inneren Kammer (200) abgelassen wird.

15. Verfahren zum Erzeugen und Aufrechterhalten einer Umgebung, um eine biologische Probe zu halten, wobei das Verfahren umfasst:
Bereitstellen eines Kammerkörpers, welcher Wände (126) aufweist, welche eine innere Kammer (200) definieren, um die biologische Probe aufzunehmen, wobei der Kammerkörper mindestens eine Basis (120) und einen Aufsatz (110) umfasst, welche ausgestaltet sind, um miteinander gekoppelt zu werden, um den Kammerkörper zu definieren;
**dadurch gekennzeichnet, dass**:
die Basis (120) und der Aufsatz (110) jeweils mehrere entsprechende in Eingriff bringbare Flansche (113, 123) umfassen, damit die Basis (120) und der Aufsatz (110) lösbar zusammen axial relativ zu einer Längsachse (A), welche die Basis (120) und den Aufsatz (110) halbiert, gekoppelt werden können durch eine Drehung der Basis (120) und/oder des Aufsatzes (110) um die Längsachse (A); wobei die Basis (120) und der Aufsatz (110) darüber hinaus jeweils eine entsprechende abdichtende Oberfläche (116M, 126M) umfassen, welche mittels einer Schnittstellen-Anpassungsanordnung über die Drehung zusammengezogen und angepasst werden kann, um eine fluiddichte Dichtung bereitzustellen, wenn die Basis (120) und der Aufsatz (110) axial zusammengekoppelt sind; Bereitstellen eines Stroms einer Flüssigkeit durch die innere Kammer (200) in Kontakt mit der biologischen Probe über einen Flüssigkeitseinlass (104IN) und einen getrennten Flüssigkeitsauslass (104OUT) an dem Kammerkörper, wobei der Flüssigkeitseinlass (104IN) und der Flüssigkeitsauslass (104OUT) an im Wesentlichen derselben axialen Höhe an der Kammerwand (126) relativ zu einer Längsachse (A) der Kammer-Anordnung, welche sich durch die Basis (120) und den Aufsatz (110) erstreckt, angeordnet sind; Bereitstellen eines Stroms von Gas durch die innere Kammer (200) in Kontakt mit der biologischen Probe über einen Gaseinlass (103IN) und einen getrennten Gasauslass (103OUT) an dem Kammerkörper;
Erzeugen einer Gas-Flüssigkeits-Schnittstelle innerhalb der inneren Kammer (200), wenn der Flüssigkeitseinlass (104IN) und der Flüssigkeitsauslass (1040UT) an einer axialen Höhe angeordnet sind, welche sich tiefer als eine axiale Höhe des Gaseinlasses (103IN) und des Gasauslasses (103OUT) relativ zu der Längsachse (A) befindet;
Anbringen der biologischen Probe an einer Position relativ zu der Längsachse (A), so dass die biologische Probe bei einem Bereich der Gas-Flüssigkeits-Schnittstelle mittels mindestens einer Probenhalterung (504) gehalten wird, welche in einem Bereich der Kammerwand (126) vorhanden ist;
wobei die axiale Position des Flüssigkeitseinlasses (104IN) und des Flüssigkeitsauslasses (104OUT) das Beibehalten der Position der Gas-Flüssigkeits-Schnittstelle innerhalb der inneren Kammer (200) relativ zu der Längsachse (A) unterstützt.

## Revendications

1. Ensemble à chambre de bioréacteur, comprenant :
au moins une base (120) et une coiffe (110) configurées pour être accouplées ensemble pour définir un corps de chambre comportant une paroi (126) qui définit une chambre interne (200) pour loger un échantillon biologique ;
**caractérisé en ce que** :
la base (120) et la coiffe (110) comprennent chacune une pluralité de brides respectives d'entrée en prise mutuelle (113, 123) pour permettre à la base (120) et à la coiffe (110) d'être, de façon libérable, accouplées et verrouillées ensemble axialement par rapport à un axe longitudinal (A) bissectant la base (120) et la coiffe (110) par rotation d'au moins une de la base (120) et de la coiffe (110) autour de l'axe longitudinal (A) ;
la base (120) et la coiffe (110) comprennent chacune en outre une surface d'étanchéité respective (116M, 126M), qui peuvent être tirées et accouplées ensemble axialement par l'intermédiaire d'un agencement à ajustement avec serrage par ladite rotation pour fournir un joint étanche aux fluides lorsque la base (120) et la coiffe (110) sont verrouillées ensemble axialement ;
une entrée de liquide (104IN) pour permettre à un liquide de s'écouler dans la chambre interne (200) ;
une sortie de liquide (104OUT) pour permettre à un liquide de sortir de la chambre interne (200) et étant séparée de l'entrée de liquide (104IN) ;
une entrée de gaz (103IN) pour permettre à un gaz de s'écouler dans la chambre interne (200) ; et
une sortie de gaz (103OUT) pour permettre à un gaz de sortir de la chambre interne (200) et étant séparée de l'entrée de gaz (103IN) ;
dans lequel l'entrée (104IN) et la sortie (104OUT) de liquide sont positionnées à une hauteur axiale plus basse qu'une hauteur axiale de l'entrée (103IN) et de la sortie (103OUT) de gaz par rapport à un axe longitudinal (A) de l'ensemble à chambre s'étendant à travers la base (120) et la coiffe (110) de sorte qu'une interface gaz-liquide puisse être créée à l'intérieur de la chambre interne entre le liquide et le gaz ;
dans lequel l'entrée (104IN) et la sortie (104OUT) de liquide sont positionnées sensiblement à la même hauteur axiale sur la paroi de chambre (126) pour aider à maintenir la position axiale de l'interface gaz-liquide ;
l'ensemble à chambre comprenant en outre au moins une monture de support d'échantillon (504) prévue dans une région de la paroi de chambre (126) pour monter l'échantillon biologique à une position par rapport à l'axe longitudinal (A) de sorte que l'échantillon biologique soit maintenable en contact avec l'interface gaz-liquide.

2. Ensemble selon la revendication 1, dans lequel la base (120) comprend une partie à pied (105) pour supporter le corps de chambre dans une orientation verticale lorsqu'elle se tient sur une structure de support.

3. Ensemble selon la revendication 1 ou 2, dans lequel la coiffe (110) comprend une fenêtre (118W) pour permettre à la chambre interne (200) d'être vue extérieurement.

4. Ensemble selon l'une quelconque des revendications précédentes, dans lequel les brides d'entrée en prise mutuelle (113, 123) font saillie radialement vers l'extérieur à partir d'au moins la base (120) et de la coiffe (110) pour permettre à la base (120) et à la coiffe (110) d'être, de façon libérable, accouplées ensemble axialement.

5. Ensemble selon la revendication 4, dans lequel les brides respectives (113, 123) s'étendent dans une direction circonférentielle autour de la base (120) et de la coiffe (110) dans lequel les brides respectives (113, 123) de la base (120) et de la coiffe (110) sont configurées pour coulisser l'une sur l'autre pour accoupler la base (120) et la coiffe (110) et empêcher la séparation axiale.

6. Ensemble selon la revendication 5, dans lequel chaque bride (113, 123) de la base (120) et de la coiffe (110) comprend des surfaces de butée respectives (113S, 123S) s'étendant dans la direction circonférentielle autour de l'axe longitudinal (A) et configurées pour être contiguës l'une à l'autre de façon coopérative lorsque la base (120) et la coiffe (110) sont accouplées ensemble par rotation autour de l'axe longitudinal (A) ;
dans lequel au moins une des surfaces de butée (113S, 123S) s'étend dans une direction circonférentielle à un angle transversal à un plan perpendiculaire à l'axe longitudinal (A) de sorte que, lorsque les brides (113, 123) de la base (120) et de la coiffe (110) sont coulissées l'une sur l'autre, la base (120) et la coiffe (110) soient tirées ensemble axialement.

7. Ensemble selon l'une quelconque des revendications précédentes, dans lequel l'entrée (103IN) et la sortie (103OUT) de gaz sont positionnées à des positions de hauteur axiale sensiblement différentes sur la paroi de chambre (126) par rapport à l'axe longitudinal (A).

8. Ensemble selon l'une quelconque des revendications précédentes, dans lequel une taille de section transversale de la sortie de liquide (104OUT) est supérieure à une taille de section transversale de l'entrée de liquide (104IN) sur la paroi de chambre (126).

9. Ensemble selon l'une quelconque des revendications précédentes, comprenant une pluralité de montures de support d'échantillon (502, 503, 504, 505) prévues à des positions axiales différentes sur le côté interne des parois de chambre (126) par rapport à l'axe longitudinal (A).

10. Ensemble selon la revendication 9, dans lequel au moins l'une des montures de support d'échantillon (502, 503, 504, 505) est positionnée dans une région supérieure de l'ensemble à chambre, lorsque l'ensemble est orienté en utilisation normale.

11. Ensemble selon les revendications 9 ou 10, dans lequel au moins une monture de support d'échantillon (504, 505) est positionnée vers une région inférieure de l'ensemble lorsque l'ensemble est orienté en utilisation normale.

12. Appareil à bioréacteur, comprenant :
un ensemble à chambre selon l'une quelconque des revendications précédentes ;
un premier réseau fluidique (606, 607, 608) accouplé en communication fluidique à l'entrée (104IN) et à la sortie (104OUT) de liquide, le premier réseau fluidique (606, 607, 608) comprenant une source de liquide (605) et une pompe (604) pour fournir un liquide à la chambre interne (200) à partir de la source de liquide (605) par l'intermédiaire de l'entrée de liquide (104IN) et pour le faire sortir de la chambre interne (200) par l'intermédiaire de la sortie de liquide (104OUT).

13. Appareil selon la revendication 12, comprenant en outre un second réseau fluidique (602, 603) accouplé en communication fluidique à l'entrée (103IN) et à la sortie (103OUT) de gaz, le second réseau fluidique (602, 603) comprenant une source de gaz (600) de sorte que du gaz puisse être fourni à la chambre interne (200) par l'intermédiaire de l'entrée de gaz (103IN) et puisse sortir de la chambre interne (200) par l'intermédiaire de la sortie de gaz (103OUT).

14. Appareil selon la revendication 13, comprenant en outre une seconde pompe (601) pour fournir un gaz à la chambre interne (200) par l'intermédiaire de l'entrée de gaz (103IN) et pour permettre à un gaz de sortir de la chambre interne (200) par l'intermédiaire de la sortie de gaz (103OUT).

15. Procédé de création et de maintien d'un environnement pour supporter une espèce biologique, le procédé comprenant :
la fourniture d'un corps de chambre comportant des parois (126) qui définissent une chambre interne (200) pour loger l'espèce biologique, le corps de chambre comprenant au moins une base (120) et une coiffe (110) configurées pour être accouplées ensemble pour définir le corps de chambre ;
**caractérisé en ce que** :
la base (120) et la coiffe (110) comprennent chacune une pluralité de brides respectives d'entrée en prise mutuelle (113, 123) pour permettre à la base (120) et à la coiffe (110) d'être, de façon libérable, accouplées ensemble axialement par rapport à un axe longitudinal (A) bissectant la base (120) et la coiffe (110) par rotation d'au moins une de la base (120) et de la coiffe (110) autour de l'axe longitudinal (A), la base (120) et la coiffe (110) comprenant chacune en outre une surface d'étanchéité respective (116M, 126M), qui peuvent être tirées et accouplées ensemble axialement par l'intermédiaire d'un agencement à ajustement avec serrage par ladite rotation pour fournir un joint étanche aux fluides lorsque la base (120) et la coiffe (110) sont accouplées ensemble axialement ;
la fourniture d'un écoulement d'un liquide à travers la chambre interne (200) en contact avec l'espèce biologique par l'intermédiaire d'une entrée de liquide (104IN) et d'une sortie de liquide séparée (104OUT) sur le corps de chambre, l'entrée (104IN) et la sortie (104OUT) de liquide étant positionnées sensiblement à la même hauteur axiale sur la paroi de chambre (126) par rapport à un axe longitudinal (A) de l'ensemble à chambre s'étendant à travers la base (120) et la coiffe (110) ;
la fourniture d'un écoulement d'un gaz à travers la chambre interne (200) en contact avec l'espèce biologique par l'intermédiaire d'une entrée de gaz (103IN) et d'une sortie de gaz séparée (103OUT) sur le corps de chambre ;
la création d'une interface gaz-liquide à l'intérieur de la chambre interne (200) lorsque l'entrée (104IN) et la sortie (104OUT) de liquide sont positionnées à une hauteur axiale plus basse qu'une hauteur axiale de l'entrée (103IN) et de la sortie (103OUT) de gaz par rapport à l'axe longitudinal (A) ;
le montage de l'espèce biologique à une position par rapport à l'axe longitudinal (A) de sorte que l'échantillon biologique soit maintenu dans une région de l'interface gaz-liquide par l'intermédiaire d'au moins une monture de support d'échantillon (504) prévue dans une région de la paroi de chambre (126) ;
dans lequel la position axiale de l'entrée (104IN) et de la sortie (104OUT) de liquide aide le maintien de la position de l'interface gaz-liquide à l'intérieur de la chambre interne (200) par rapport à l'axe longitudinal (A).
